(19) Europäisches Patentamt
European Patent Office
Office européen des brevets

(11) EP 4 048 709 B1

(12) EUROPEAN PATENT SPECIFICATION

(45) Date of publication and mention of the grant of the patent:
**26.02.2025 Bulletin 2025/09**

(21) Application number: **21840418.4**

(22) Date of filing: **31.05.2021**

(51) International Patent Classification (IPC):
***C08F 8/42*** (2006.01) ***C09B 47/00*** (2006.01)
***C09B 47/04*** (2006.01) ***C09B 47/08*** (2006.01)
***A61K 31/555*** (2006.01) ***A61P 35/00*** (2006.01)
***A61K 41/00*** (2020.01)

(52) Cooperative Patent Classification (CPC):
**A61K 31/555; A61K 41/0071; A61P 35/00**

(86) International application number:
**PCT/TR2021/050515**

(87) International publication number:
**WO 2022/146292 (07.07.2022 Gazette 2022/27)**

(54) **A DRUG FOR THE TREATMENT OF CERVICAL CANCER**
ARZNEIMITTEL ZUR BEHANDLUNG VON GEBÄRMUTTERHALSKREBS
MÉDICAMENT POUR LE TRAITEMENT DU CANCER DU COL DE L'UTÉRUS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.12.2020 TR 202022158**

(43) Date of publication of application:
**31.08.2022 Bulletin 2022/35**

(73) Proprietor: **Yildiz Teknik Universitesi**
**34220 Istanbul (TR)**

(72) Inventors:
- **CANLICA, Mevlude**
**Instanbul (TR)**
- **TOPCUL, Mehmet Rifki**
**Instanbul (TR)**
- **CETIN, Idil**
**Instanbul (TR)**

(74) Representative: **Sevinç, Cenk**
**Grup Ofis Marka Patent A.S.**
**Atatürk Bulvari, 211/11**
**Kavaklidere**
**06680 Ankara (TR)**

(56) References cited:
**JP-A- H0 959 529**

- **KOÇAN HALIT ET AL: "Photophysicochemical, calf thymus DNA binding and in vitro photocytotoxicity properties of tetra-morpholinoethoxy-substituted phthalocyanines and their water-soluble quaternized derivatives", JBIC. JOURNAL OF BIOLOGICAL INORGANIC CHEMISTRY, SPRINGER, DE, vol. 22, no. 8, 19 October 2017 (2017-10-19), pages 1251 - 1266, XP036353036, ISSN: 0949-8257, [retrieved on 20171019], DOI: 10.1007/S00775-017-1499-3**
- **CANLICA, MEVLÜDE ; TOPÇUL, MEHMET RIFKI ; ÇETIN, İDIL: "In vitro antiproliferative effect of four ball-type phthalocyanines linked by t-butylcatechol and high singlet oxygen production", JOURNAL OF COORDINATION CHEMISTRY, vol. 73, no. 23, 1 December 2020 (2020-12-01), London , pages 3291 - 3305, XP009538647, ISSN: 0095-8972, DOI: 10.1080/00958972.2020.1845320**
- **NJEMUWA NWAJI, JOHN MACK, TEBELLO NYOKONG: "Enhanced nonlinear optical response of benzothiazole substituted ball-type phthalocyanines in the presence of metallic nanoparticles", OPTICAL MATERIALS, vol. 82, 29 May 2018 (2018-05-29), pages 93 - 103, XP055954740**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- ALLEN CYNTHIA M., SHARMAN WESLEY M., VAN LIER JOHAN E.: "Current status of phthalocyanines in the photodynamic therapy of cancer", JOURNAL OF PORPHYRINS AND PHTHALOCYANINES, BOGNOR REGIS, GB, vol. 05, no. 02, 17 February 2001 (2001-02-17), GB , pages 161 - 169, XP055941770, ISSN: 1088-4246, DOI: 10.1002/jpp.324

## Description

### Technical Field of the Invention

The invention relates to a compound named indium (III) phthalocyanine [1,8(11),15(18),22(25)-tetra-(3,5-di-tert-butylphenoxy) indium (III) phthalocyanine] for the treatment of cervical cancer, as defined in claim 1. This compound is applied in photodynamic therapy (PDT), a method that uses a photosensitizing agent activated by light to produce reactive oxygen species capable of destroying cancer cells. In particular, the invention shows that this compound effectively reduces the viability of HeLa cells, a type of human cervical carcinoma cell, by causing cell death by various mechanisms depending on the concentration used.

### State of the Art

Cancer is abnormal cell growth that is caused by multiple changes in gene expression, which is defined as a phenomenon involving nearly a hundred complex diseases exhibiting different behaviors depending on the cell type they originate from. It disrupts the balance between cell proliferation and cell death. It may spread to various regions of the body due to its ability of invasion and metastasis, and cause tissue and organs to lose their function in the region where they spread. Cancers vary by their age of onset, growth rate, spread, stage, and response to treatment. However, all cancer types have common characteristics at the molecular level which unite all cancer types in a single unit family. Cancer, if not treated, generally results in the death of people, and today, the second leading cause of death worldwide is cancer-related deaths. Death caused by cancer can occur as a result of the side effects of existing conventional treatments as well as the damage caused by the disease. Patients cannot be administered sufficient doses in order to avoid the fatal results which may be induced by the side effects of the conventional drugs and as a result, complete and effective treatment cannot be provided. The disease may relapse due to the fact that all cells in the tumor population cannot be killed even in cases where the disease is thought to be cured.

Today, three main treatment methods are implemented in cancer treatment, which include surgery, chemotherapy, and radiotherapy. Less frequently, hormonal therapies, biological therapies, and targeted therapies are also employed. Said treatment methods may be implemented individually or in combination. In the surgical method, cancerous tissue is removed from the body. The surgical method is the first method applied in many types of cancer. Radiotherapy is a cancer treatment method in which high-energy X-rays or other types of radiation are used in order to kill cancerous cells or prevent their growth. This treatment method is divided into two types as external radiation and internal radiation. In the external radiation method, X-rays are directed from a machine directly to the cancerous cell and surrounding tissues. In internal radiation, capsules that contain radioactive material are placed into the body cavity of a patient, on and around the tumor. Some patients experience side effects such as fatigue, skin rash, and nausea after radiotherapy. Chemotherapy is a cancer treatment method that is applied in order to prevent tumor cell growth by either killing cancerous cells or stopping their division. Chemotherapy, as another cancer treatment method, is performed in order to destroy cancer cells or to control the growth of said cells by means of using anticancer drugs. Chemotherapy can be administered to the patient in different ways. The drugs enter the bloodstream and may reach cancerous cells in the body (systemic chemotherapy) when chemotherapy is administered orally or injected into a vein or muscle. When chemotherapeutic drugs are placed directly in the cerebrospinal fluid, an organ, or a body cavity such as the abdomen, they mainly affect cancer cells in said areas (regional chemotherapy). The method of administration of chemotherapy depends on the type and stage of cancer that is being treated. Chemotherapeutic drugs stop cell division by inhibiting enzymes involved in DNA replication or metabolism. However, chemotherapeutic drugs do not specifically target tumor cells, but also damage normally dividing cells of rapidly regenerating tissues such as bone marrow, intestinal mucosa, and hair follicles. Therefore, chemotherapy brings many problems along. Cancer cells and rapidly dividing normal cells are difficult to distinguish from each other due to their similarity, and cells may also develop resistance to drugs. Every drug cannot be applied to every patient since every person and tumor is genetically different. For this reason, some cells treated with chemotherapy can survive and turn into drug-resistant cancer cells, and even treatment-related diseases may develop afterwards. In addition, many of the drugs that kill tumors can also cause mutations that turn normal cells into cancerous cells. Consequently, all of the aforementioned reasons necessitate developing new drugs that target cancer cells but do not affect normal cells.

Cervical cancer is one of the most common cancer types among women, in which 98% of said cancer is caused by HPV (Human Papillomavirus). It occurs as a result of the proliferation of cancer cells in the cervix that connects the uterus and the genital tract (vagina). Cervical cancer is a type of cancer that usually develops slowly. Cervical cells go through changes called dysplasia before cancer appears in the cervix. In cervical cancer, abnormal cells begin to appear in the cervical tissue and may become cancerous cells over time, and said abnormal cells begin growing and spreading more extensively in the cervix and surrounding areas. In some cases, the fact that being unable to provide a complete treatment for cervical cancer with the treatments available in the state of the art may result in a relapse of the disease just like in other types of cancer.

There are various treatment options for patients with have cervical cancer. As in other types of cancer, standard treatment options include surgery, radiotherapy, and chemotherapy. An early diagnosis is the most effective way of the treatment process. In the surgical treatment method, some part or all of the cancerous tissue in the cervix can be removed by means of administering general anesthesia to the patient. Chemotherapy or radiotherapy methods are applied to patients after surgical intervention if considered necessary. In chemotherapy, it is required to search for alternative short-term and effective treatment methods since drugs that are not related to cancer but used against cancer cause deaths due to side effects (heart attack, etc.) thereof, and cause reduced quality of life.

When compared to other treatment methods employed nowadays, the photodynamic therapy (PDT) method is a less harmful and effective cancer treatment method, which is performed in the existence of photosensitizer (photosensitive substance), light and oxygen molecules. Said photosensitizers are defined as chemical compounds that absorb the energy of light with a wavelength that suits their structure, that transform from the ground state to the excited state, and that transfer the energy they absorb into biomolecules. The first example of photodynamic therapy was recorded in 1900 when Raab has observed a toxic effect as a result of reacting the acridine orange with light. In addition, Raab's study has shown that it is possible to kill paramecium, which is a unicellular animal species, by means of using acridine dye and sunlight. When the same experiment was repeated in an environment where there was no light, it was revealed that the dye has interacted with the light since no change was observed (Rasmussen-Taxdal, 1955). In 1905, photodynamic therapy was applied to malignant tumors in facial basal cells on 6 patients by Tappeiner and Jesionek. In this study, 1% eosin solution was used, and sunlight and arc lamps were utilized for a long period of time, and as a result of the studies conducted, and improvement was observed in tumors of four patients (Tappeiner, 1909). The first steps of modern photodynamic therapy were taken by Lipson et al. through their studies regarding hematoporphyrin derivative (HPD) in 1961, and an important step was taken in photodynamic therapy when Parker et al. have achieved regression in experimental iris neovascularization using the hematoporphyrin derivative in 1984. In 1987, Dougherty et al. conducted research on 113 skin and subcutaneous tumors and as a result of these studies, partial or complete recovery was observed in 111 tumors. However, none of these studies were put into clinical practice. In PDT applications, a photosensitive substance called a photosensitizer, which does not have a toxic effect individually, is exposed to light. The resulting free radicals and singlet oxygen interact with many biological molecules and cause the death of cancerous cells through apoptosis (programmed cell death) or necrosis. In other words, PDT is based on a principle in which the drug accumulates in the tumor tissue and destroys the tumor by being stimulated with a light source of a certain wavelength after the photosensitive drug is administered to the patient through intravenous route. Said photosensitive drugs have a much higher tendency to accumulate and be retained in tumor tissues compared to normal tissues. Light may stay longer in tumor tissue compared to normal tissue. Singlet oxygen, which is released as a result of occurring reactions, is extremely toxic when the light is absorbed by the photosensitive drug. Singlet oxygen causes necrosis in the desired region only and although its life in the tissue is noticeably short, it is local. Thus, the cells in the tumor region are destroyed without damaging the healthy tissues.

In photodynamic therapy, light energy is converted into chemical energy by means of a non-toxic photosensitive substance, the photosensitizer, and said chemical energy is transferred to the target tissue. In photodynamic therapy, the administration of the photosensitizer into the body consists of two basic steps, including the production of a photochemical effect in the targeted area and the activation of said substance. Basically, there are two selectivity features. The first feature is that the photosensitizer, which is a light-sensitive substance administered to the body, is accumulated especially in the target region, and the other is that the light activating said substance is applied only to the desired region. The mechanism of action of photodynamic therapy occurs such that the photosensitive substance, which is in the ground state, becomes a triplet with high energy after being excited by a light source of a wavelength that it can absorb, and activated photosensitizers follow two different paths, which are Type I and Type II reactions, so as to interact with biomolecules. One of the three essential elements in photodynamic therapy is oxygen, and the importance of oxygen is stemming from its transition to singlet oxygen form. There are two unpaired electrons in the outer antibonding orbitals when oxygen is in the ground state. The singlet oxygen, which is formed as a result of changing the direction of one of said outer electrons, is highly reactive. Singlet oxygen interacts with other molecules and transfers its energy due to its high reactivity. The creation of singlet oxygen constitutes the basis of the PDT method.

Type I reactions create electron transfer reactions which lead to the formation of ROS (Reactive Oxygen Species). Type II reactions are energy transfer reactions that cause ($^1O_2$) formation. In Type I reactions, radicals with highly reactive properties appear when the hydrogen atom of the excited photosensitizer is transferred to a molecule in the cell and said radicals react with molecular oxygen. As a result, oxygenated products are formed. Several photosensitive substances used in photosensitive substances show their effects through Type II reaction mechanisms over $^1O_2$, which is ROS most of the time. In Type II reactions, the excited photosensitizer gives its energy directly to molecular oxygen ($O_2$) and induces the formation of singlet oxygen ($^1O_2$). Singlet oxygen ($^1O_2$), which is the electronically excited state of the molecular oxygen, oxidizes the biomolecules and causes damage in the cell. Type III reactions are immune complex related reactions. These reactions are also known as hypersensitivity reactions. Type III reaction occurs when the antibody against a soluble antigen binds with the antigen and activates the complex system. An immune complex is formed as a result of antigen-antibody binding. The intensity of the reaction is associated with the size and distribution of the immune

complex. Immune complexes are observed quite frequently in the blood vessel wall, the synovial membrane of the joints, and the choroidal plexus of the brain. Type III reactions may be local or systemic. Type IV reactions are known as cell-mediated or delayed-type hypersensitivity. The conventional example of these reactions is the tuberculin (Mantoux) reaction, which peaks 48 hours after antigen injection. Type IV reactions are associated with many autoimmune and infectious diseases, foreign antigens, and infection-induced granuloma pathogenesis.

The wavelength of the light used in photodynamic therapy is particularly important in terms of the depth that the light may reach in the tissue. However, the wavelength and the energy of the light used are inversely proportional. Therefore, the use of a light source with high wavelength should be preferred in order to minimize the side effects of the therapy that is being administered. It has been determined that the optimal wavelength range is between 650 and 850 nm for PDT applications, and said range is defined as 'Phototherapeutic window'. Arc lamps are utilized to stimulate photosensitizers since they are easy to use and inexpensive. However, UV and IR filters must be used in order to prevent heating since such lamps are light sources with a broad spectrum. Additionally, LEDs with a wide wavelength range may also be used as a light source in PDT. Another important light source is laser light that may be emitted in a single color and in synchronized light waves with little deviation, which facilitates focusing the light rays. Lasers have characteristic features such as the operation of optical fibers in photodynamic therapy, the treatment of endoscopic internal tumors, and the placement of a light source in tumor tissue with respect to their use in photodynamic therapy. Moreover, one of the reasons to use the laser light for the activation of the photosensitizer in PTD is that the laser light has high power.

The photosensitizer used in the region of the body, which is threatened and damaged by cancer cells may vary based on the application of the light and photosensitizer agent that activates it. The type of photosensitizer agent also varies according to the light source and the type of treatment applied. After the photosensitizer agent used in the treatment is received by cancer cells, the light with a wavelength, which can be absorbed by the agent, is applied only to the part with cancerous cells. Photodynamic therapy is an important treatment method for inducing damage to the blood vessels feeding cancer cells and enabling the immune system to attack cancer cells. The time period between the administration of the photosensitizer agent and the application of light to the patient varies depending on the photosensitizer agent used. Photosensitizer to be used is determined based on characteristics such as; accumulation in tumor tissues, accumulation in the desired region in a short period of time, not containing any impurities, not getting accumulating in healthy tissues, not having any toxic effects in case there is no stimulating light, and tending to form singlet oxygen with high efficiency.

Phthalocyanines structures of which do not deteriorate when exposed to heat, light, acids, and bases, were discovered by Braun and Tchemiac in 1907 in a study conducted for obtaining o-cyanobenzamide from phthalimide and acetic acid, however, it was the study conducted by Linstead and Robertson which shed light upon the structures thereof between the years of 1933-1940. Phthalocyanines are a class of compounds consisting of a 16-membered planar macro ring having a highly conjugated 18 $\pi$ electron system. Phthalocyanines are not found in nature although they are structurally similar to porphyrins such as hemoglobin, chlorophyll a, and B12. Today, phthalocyanines with the characteristics of blue and green colors are used in applications such as lasers, sensors, nanotubes, self-emitting compounds (OLED), photodynamic therapy (PDT), cancer treatment, and catalysts, in addition to their use as pigments in printing inks, coatings, paints, and plastics.

The study of Kocan et al. can be given as an example of phthalocyanins in the state of the art. This study synthesized morpholinoethoxy-substituted metal-free (3), zinc(II) (4), and indium(III) (5) phthalocyanines, which were then converted to water-soluble quaternized derivatives (3Q-5Q) using methyl iodide. However, the high amount of N,N-Dimethylmethanamide (DMF) solvent used during the synthesis of this material does not have enough environmentally friendly properties

The reasons such as insufficient treatment methods used in cervical cancer treatment in the state of the art, side effects caused by chemotherapeutic drugs, deaths stemming from side effects (heart attack, etc.) necessitated developing a drug for use in the treatment of cervical cancer.

## Brief Description and Objects of the Invention

The substance called indium (III) phthalocyanine for the treatment of cervical cancer is the subject of the invention. The full name of the compound subject to the invention is 1,8(11),15(18),22(25)-tetra-(3,5-di-tert-butylphenoxy) indium (III) phthalocyanine. In the present invention, 335In(III)Pc compound was applied to HeLa cells originating from human cervical carcinoma by means of employing the PDT method, and it was demonstrated that the cell viability decreased depending on the dose as a result of the proliferation rate analysis and that 100 $\mu$M of product concentration cause the survival of 47.45 % of the cells, in other words, the death of 52.55 % of the cells. An evaluation of cell index parameters demonstrated that different concentrations both show antiproliferative effects on cells and lead to cell death through various mechanisms. It has been demonstrated that the cells undergo mitosis division decreased significantly depending on time by means of the mitosis index parameter. As such, it has been found that it leads to the death of cancer cells by using various mechanisms in different concentrations.

The most important object of the present invention is to ensure that cervical cancer is treated effectively. In the

present invention, more than half of HeLa cancer cells are killed by means of the indium (III) phthalocyanine compound, which is the active ingredient that is being synthesized.

Another object of the present invention is to produce a drug with an anticarcinogenic effect for use in cervical cancer treatment by means of the photodynamic therapy method. A drug that contains indium (III) phthalocyanine compound ensures that the cancerous cells causing cervical cancer are killed and cancer is treated. The IC50 concentration of indium (III) phthalocyanine compound applied to HeLa cells, which is a cervical cancer strain, was determined as 100 $\mu$M.

Another object of the present invention is to obtain an anticancer drug by means of an eco-friendly synthesis method. In the present invention, no solvent is used in the synthesis of indium (III) phthalocyanine compound, which is the active ingredient of the drug synthesized for use in cervical cancer treatment. Thus, it is eco-friendly within the scope of green synthesis.

Yet another object of the present invention is to obtain an active ingredient that has anticancer effects and high singlet oxygen production for being used in photodynamic therapy for the treatment of cervical cancer. The use of indium (III) phthalocyanine compound in photodynamic therapy produces exceedingly high amounts of singlet oxygen.

**Description of the Figures**

**FIGURE 1** illustrates the chemical structures of 3-(3,5-di-tert-butylphenoxy) phthalonitrile (Compound 1) and 11,8(11), 15(18),22(25)-tetra-(3,5-di-tert-butylphenoxy) In(III)phthalocyanine (Compound 2) compounds.

**FIGURE 2** illustrates the FT-IR spectrum of Compound 1.

**FIGURE 3** illustrates the FT-IR spectrum of Compound 2.

**FIGURE 4** illustrates the NMR spectrum of Compound 2.

**FIGURE 5** illustrates the mass spectrum of Compound 2.

**FIGURE 6** illustrates the fluorescence spectrum of Compound 2.

**FIGURE 7** illustrates the absorbance chart of Compound 2 under the light.

**FIGURE 8** illustrates the photodegradation spectrum of Compound 2 under the light.

**Detailed Description of the Invention**

The present invention relates to a 1,8(11), 15(18),22(25)-tetra-(3,5-di-tert-butylphenoxy) indium (III) phthalocyanine (Compound 2, In(III)Pc or 335In(III)Pc) compound in order to be applied with photodynamic therapy for use in the treatment of cervical cancer. The present invention further relates to an anticarcinogenic drug in which the 1,8(11),15(18),22(25)-tetra-(3,5-di-tert-butylphenoxy) indium (III) phthalocyanine compound is used as an active ingredient. The chemical structure of Compound 2 is as shown in Formula 1:

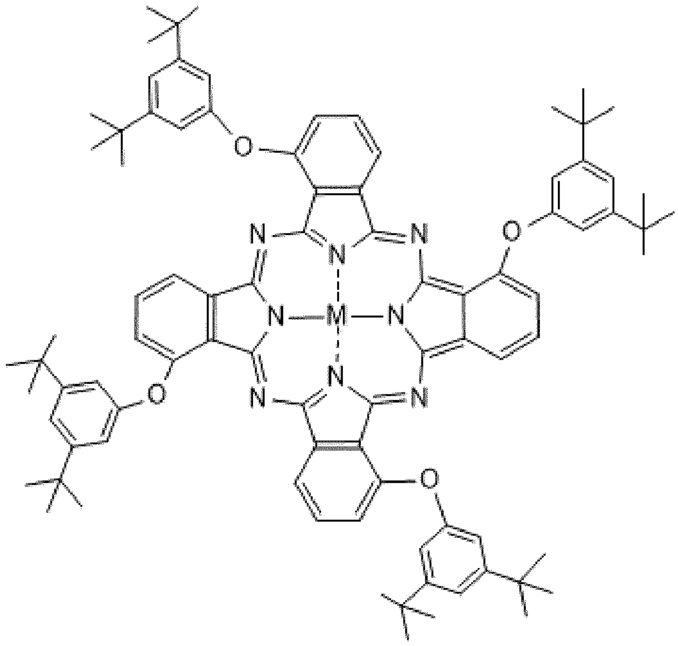

## Formula 1

Synthesis method of said indium (III) phthalocyanine compound comprises the process steps of;

- Powdering 0,508 g of 3-(3,5-di-tert-butylphenoxy) phthalonitrile compound (Compound I), and 0,101 g of indium (III) acetate inside a mortar,
- Filling the powdered mixture into the reaction tube,
- Adding ten drops of 1,8-Diazabicyclo[5.4.0]undec-7-ene (DBU) to the reaction tube and passing through Argon gas for 5 minutes,
- Heating it in a fume cupboard at 360°C after the reaction vessel is tightly closed,
- Continuing the heating for 8 more minutes after the color turns green,
- Checking its purity by means of the thin-layer chromatography (TLC) after cooling it to room temperature, and purifying the green product obtained as a result by means of column chromatography with chloroform,
- Drying the purified 1,8(11),15(18),22(25)-tetra-(3,5-di-tert-butylphenoxy) indium (III) phthalocyanine (Compound 2) by using phosphorus pentoxide ($P_2O_5$) in vacuum for one week.

The compound obtained by means of the aforementioned method is in the form of a dark green crystal, and the yield thereof is 82 mg (16,14%). The closed formula of Compound 2, which has a melting point >300°C is $C_{89}H_{99}InN_8O_6$, and its molecular weight is 1491,61 g/mol. It has been determined that C, 71.66; H, 6.69; 7.70; N, 7.51; O, 6.44 when the theoretical elemental analysis of the compound was performed. FTIR analysis was conducted in order to shed light upon the structure of said Compound 2 and it is illustrated in Figure 3. FTIR results have indicated that the Compound 2 structure was synthesized correctly. Said compound is capable of getting dissolved in common solvents such as chloroform (CHCl3), acetone (C3H6O), tetrahydrofuran (THF), ethanol (C2H5OH), methanol (CH3OH), dimethylformamide (DMF), or dimethyl sulfoxide (DMSO) (Table 1).

**Table 1.** The solubility data of the In(III) Pc compound.

| Complex | Solvents and Dielectric constants (ε: F/m) | | | | | | |
|---|---|---|---|---|---|---|---|
| In(III) Pc | Chloroform (4,8) | THF (7,4) | Acetone (20,7) | Ethanol (24,3) | Methanol (32,6) | DMF (36,7) | DMSO (45,0) |
| Q-Band (λmax, nm) | 725 | 718 | 714 | 712 | 713 | 709 | 710 |
| Vibration Band (λmax, nm) | 651 | 646 | 643 | 643 | 642 | 640 | 639 |
| B-Band (λmax, nm) | 333 | 326 | 364 | 328 | 331 | 330 | 337 |

Equation-1 was used in order to calculate the fluorescence quantum yield (ΦF) of Compound 2. Hence, approximately 10-6 molar solution of Compound 2 complex and standard Zinc (II) phthalocyanine (ZnPc) in dimethyl sulfoxide (DMSO) was prepared, 639 nm was chosen as the most suitable excitation wavelength in order to form the fluorescence spectrum. Thus, emission and excitation spectra were obtained. Equation-1 is as follows:

$$\Phi_F = \Phi_{F(Std)} \frac{F \,.\, A_{Std} \,.\, n^2}{F_{Std} \,.\, A \,.\, n_{Std}^2}$$

wherein,

ΦF; the fluorescence quantum yield of the sample,
ΦF(std); the fluorescence quantum yield of the standard compound,
F; the area under the fluorescence emission curve of the sample,
$F_{Std}$; the area under the fluorescence emission curve of the standard compound,
A; the absorbance of sample,
$A_{std}$; the absorbance of the standard compound,
η; the refractive index of the solvent in which the sample is dissolved,
$\eta_{Std}$; the refractive index of the solvent in which the standard compound is dissolved.

Singlet oxygen quantum yields of the inventive Compound 2 were calculated by means of Zinc (II) phthalocyanine (ZnPc) standard and 1,3-diphenylisobenzofuran (DPBF) singlet oxygen quencher at room conditions via using Equation-2 below:

$$\Phi_\Delta = \Phi_\Delta^{Std} \cdot \frac{R_{DPBF} \cdot I_{abs}^{Std}}{R_{DPBF}^{Std} \cdot I_{abs}}$$

wherein,

$\Phi^{Std}_\Delta$; the singlet oxygen yield (ZnPc ΦΔ = 0,53 in THF)
ΦΔ; for example, singlet oxygen yield,
$R_{DPBF}$; the degradation rate of DPBF in the presence of the sample,
$R_{OPBFstd}$; the degradation rate of DPBF in the presence of the standard,
$I_{abs}$; the light absorption rate of sample and standard,
$I_{abStd}$; the light absorption rate of the standard.

DPBF with a concentration of 3 $\times 10^5$ mol.$L^{-1}$ was used in order to prevent chain reactions caused by the presence of singlet oxygen. Solutions of a sensitizer containing DPBF (absorbance at irradiation wavelength = 2.0) were prepared in a dark environment and irradiated in the Q band area by means of using the setup described above. This yield was found as 49%, according to Figure 7. The maximum absorbance of the Q band in the solutions of all complexes was set to 1.0 in the solvent used in order to determine the photodegradation quantum yield (Φd), and the decrease in the intensity

of the Q band under irradiation with time is observed by means of using the following Equation-3:

$$\Phi_d = \frac{(C_0 - C_t).V.N_A}{I_{abs}.S.t}$$

wherein,

$C_0$ (mol.dm$^{-3}$); concentration of Compound 2 before irradiation,
Ct (mol.dm$^{-3}$); concentration of Compound 2 before irradiation,
V; reaction volume (3.0 cm$^3$),
S; irradiated cell area,
t; irradiation time,
$N_A$; Avogadro's number,
$I_{abs}$; the overlapping integration of the radiation source intensity. Accordingly, the photodegradation quantum yield was found as ($\Phi$d) = 2,0017$\times$10$^{-05}$, and this result is an acceptable value for phthalocyanine compounds and indicates that the In(III)Pc complex is stable under the light. It has been observed that Compound 2 is active in destroying cancer cells, and as such may have a potential for the treatment of cancer with PDT when considering the PDT characteristic measurement results described above.

Different concentrations of In(III)Pc complex were applied to cultured human cervical cancer originated HeLa cells for 24 hours in order to determine the changes in mitochondrial dehydrogenase enzyme activity of cancer cells by means of the In(III)Pc complex obtained. For proliferation rate analysis, routinely cultured HeLa cells were cultivated in culture plates with 96 wells such that the growth medium is 3$\times$10$^4$ cell/ml per well. In(III)Pc complex prepared at 50 $\mu$M, 100 $\mu$M, and 150 $\mu$M concentrations were applied to the cells after waiting for the cells to adhere and spread. The growth medium in the wells was withdrawn and 40 $\mu$l of 3-(4,5-dimethylthiazol-2-yl)-2,5-diphenyltetrazolium bromide (MTT) (5mg/ml) was added at the end of the experiment periods. 160 $\mu$l of dimethyl sulfoxide (DMSO) was added to the wells with MTT and incubated for 1 hour in a shaker incubator after waiting for approximately 4 hours. The absorbance values of the formed experimental groups were carried out by means of the spectrophotometer measurements at 570 nm with reference to the 690 nm wavelength after the formazan crystals formed are dissolved for the 1 hour incubation period. The absorbance values obtained as a result of this application were measured as 590$\times$10$^{-3}$ for the control group; 460 $\times$10$^{-3}$ for 50 $\mu$M; 280 $\times$10$^{-3}$ for 100 $\mu$M, and 170 $\times$10$^{-3}$ for 150 $\mu$M. According to the control group, which is considered 100% for HeLa cells, it was observed that 50 $\mu$M of Compound 2 concentration reduced the viability of cells to 78% that 100 $\mu$M of Compound 2 concentration increases the viability of cells to 47.45%, and 150 $\mu$M of Compound 2 concentration reduced the viability of cells to 28.81 %.

In the present invention, the cell index parameter was evaluated by means of using a real-time cell analysis system in order to both support the results obtained and to determine the types of cell death, and an E-Plate with 16 wells was used in order to evaluate this parameter. Primarily, 100 $\mu$l of growth medium was added to each well of the E-Plate, and background measurements were taken in the cell analyzer device. Then, cultivation is performed in each well such that the 100 $\mu$l of growth medium has 6000 cells for HeLa cells. The E-Plates were incubated for 20 minutes in a sterile working cabinet at room temperature, and then placed in the cell analyzer device and incubation was continued at 37°C under ambient conditions of 5% carbon dioxide (CO2) after the cultivation process. The cell analyzer was issued a command to take the measurement every 15 minutes after the cell cultivation to the E-Plates was performed. After approximately 24 hours of cell cultivation, the growth medium in the E-Plates was changed with a growth medium containing different concentrations of In(III)Pc complex at the 1/3rd slice of the proliferation phase of the cells, and measurements were continued at 15-minute intervals for 72 hours. Subsequently, graphics of concentration and time-dependent cell index values were transferred to the computer screen. The antiproliferative effects of the In(III)Pc complex on HeLa cells are observed when the cell index values obtained from the real-time cell analysis system are examined. The 50 $\mu$M of In(III)Pc complex applied does not show a significant effect when the cell index values obtained are compared with the standard curves, however, 150 $\mu$M of In(III)Pc complex concentration produces a cytoskeletal effect, while 100 $\mu$M of In(III)Pc complex concentration, which is an optimum concentration, is causing DNA damage on cells.

As a result of applying the optimum concentration of the product(100 $\mu$M) on HeLa cells for 0-72 hours, the preparations prepared according to the experimental groups were primarily hydrolyzed with 1 N hydrochloric acid (HCl) at room temperature for 1 minute and then with 1 N HCl at 60°C for 10 minutes in order to determine the effects on the mitotic index values of the cells. After hydrolysis, the Feulgen method was applied to the preparations for 1 hour. Then, these preparations were washed 3 times for 2 minutes with a washing solution, which is specific to the Feulgen method, and air-dried. After the preparations were dried, they were prepared for counting by means of staining with Giemsa dye for 2

minutes. Late prophase, metaphase, anaphase, and telophase stages were counted in order to determine the mitosis index (MI) values of said preparations. The early prophase stage was evaluated together with this stage, as it was morphologically similar to the cells in the interphase group.

Three preparations were evaluated for each experimental group and mitosis index values were determined by means of counting 3000 cells on average from each preparation. The mitosis index values have been decreased from 6% to 3,7% at the 24th hour; from 6,68% to 2,21% at the 48th hour, and from 7,56% to 1,19% at the 72nd hour.

The values of cell kinetic parameters determined according to concentration and time applied in all experimental groups were evaluated according to control and each other, and to that end, a one-way ANOVA test was applied to the values determined from all experimental groups. The significances of the groups according to the control were evaluated with the DUNNETT'S test, and the significances of the groups with each other were evaluated with the t-test. A significance level of $p<0.01$ was selected as a basis in statistical evaluations. The anticancer effects of Compound 2 in HeLa cell cultures originated from human cervical cancer were investigated by means of evaluating various cell kinetic parameters. The data obtained has indicated that Compound 2 caused a significant decrease in cell proliferation depending on the concentration and time, and at the same time, cell death has occurred with different death mechanisms at different concentrations. 100 μM of Compound 2 concentration had a DNA damaging effect on cells while 150 μM of Compound 2 concentration had a cytoskeletal effect. In addition, the mitosis index parameter using 100 μM of Compound 2 concentration, which is the optimum concentration, indicates that mitosis cell division decreases significantly over time. Consequently, the significant death of human cervical cancer cells via different mechanisms indicates that the inventive compound may be a drug that can be used in the treatment of this cancer.

In the second stage, after administering the 335In(II)Pc substance to the body, only the cancerous region is exposed to red light, and therefore, the treatment of cervical cancer is provided effectively.

**REFERENCES**

Klug, W.S., Cummings, M.R., Spencer, C.A., 2006, Concepts of Genetics, Pearson, UK, 0-13191883-8.

Pitot HC, Loeb DD (2002). Some basic and applied principles of cancer chemotherapy. In 'Fundamentals of Oncology', Eds. Pitot HC, Loeb DD (2002). Marcel Dekker Inc, New York, pp 901-945.

Wu HC, Huang CT, Chang DK (2008). Anti-angiogenic therapeutic drugs for treatment of human cancer. J Cancer Molecules, 4, 37-45.

Aqeilan RI, Zanesi N, Croce CM (2009). Environmental, genetic, and viral causes of cancer. In The biology and treatment of cancer', Eds. Pardee AB, Stein GS. John Wiley & Sons, Inc, New Jersey, pp 35-56.

M. Brewis, G.J. Clarkson, P. Humberstone, S. Makhseed, N.B. McKeown, The synthesis of some phthalocyanines and napthalocyanines derived from sterically hindered phenols, Chem. Eur. J. 4 (1998) 1633-1640.

S. Fery-Forgues, D. J. Lavabre, Are fluorescence quantum yields so tricky to measure? A demonstration using familiar stationery products, Chem. Ed. 76 (1999) 1260-1264.

J. Fu, X.Y. Li, D.K.P. Ng, C. Wu, Encapsulation of phthalocyanines in biodegradable poly (sebacic anhydride) nanoparticles, Langmuir. Ed. 18 (2002) 3843-3847.

A. Ogunsipe, A.D. Maree, T.J. Nyokong, Solvent effects on the photochemical and fluorescence properties of zinc phthalocyanine derivatives. Mol Struct. 650 (2003) 131-140.

A. Ogunsipe, J.Y. Chen, T. Nyokong, Photophysical and photochemical studies of zinc (II) phthalocyanine derivatives-effects of substituents and solvents, New J. Chem. 28 (2004) 822-827.

D. Wohrle, G. Schnurpfeil, S.G. Makarov, A. Kazarin, O.N. Suvorovab, Practical applications of phthalocyanines-from dyes and pigments to materials for optical, electronic and photo-electronic devices, Macroheterocycles. 5 (2012) 191-202.

M.O. Liua, C. Taib, M. Saina, A. Teh Hua, F. Chouc, Photodynamic applications of phthalocyanines, J. Photochem. Photobiol. A Chem. 165 (2004) 131-136.

I. J. Macdonald, T.J. Dougherty, Basic principles of photodynamic therapy, J. Porphyrins Phthalocyan. 5 (2001) 105-129.

J. E. van Lier, N. Brasseur, B. Paquette, J. R. Wagner, H. Ali, R. Langlois, J. Rousseau, Phthalocyanines as Sensitizers for Photodynamic Therapy of Cancer, NATO ASI Series, Vol. H15

Shawn Swavey and Matthew Tran, Porphyrin and Phthalocyanine Photosensitizers as PDT Agents: A New Modality for the Treatment of Melanoma, Recent Advances in the Biology, Therapy and Management of Melanoma, DOI: 10.5772/54940, 2013.

Vrouenraets MB, Visser GW, Snow GB, van Dongen GA. Basic principles, applications in oncology and improved selectivity of photodynamic therapy. Anticancer Research 2003; 23(1 B):505-522.

Dolmans DE, Fukumura D, Jain RK. Photodynamic therapy for cancer. Nature Reviews Cancer 2003; 3(5):380-387.

Hodgkinson N, Kruger CA, Mokwena M, Abrahamse H. Cervical cancer cells (HeLa) response to photodynamic therapy using a zinc phthalocyanine photosensitizer. Journal of Photochemistry & Photobiology, B: Biology 2017; 177: 32-38.

Guan D, Guo Q, Chen L, Wu S, Nan F, Zhang Y. Study on application of photodynamic therapy for the treatment of cervical cancer. Int J Clin Exp Med 2016;9(12):23337- 23344

Li W, Tan G, Cheng J, Zhao L, Wang Z, Jin Y. A Novel Photosensitizer 31,131-phenylhydrazine -Mppa (BPHM) and Its in Vitro Photodynamic Therapy against HeLa Cells. Molecules 2016; 21(5): 558

Mascaraque M, Delgado-Wicke P, Damian A, Lucena SR, Carrasco E, Juarranz A. Mitotic Catastrophe Induced in HeLa Tumor Cells by Photodynamic Therapy with Methyl-aminolevulinate. Int. J. Mol. Sci. 2019; 20: 1229

Kocan, H., Kaya, K., Ozcesmeci, I. et al. Photophysicochemical, calf thymus DNA binding and in vitro photocytotoxicity properties of tetra-morpholinoethoxy-substituted phthalocyanines and their water-soluble quaternized derivatives. J Biol Inorg Chem 22, 1251-1266 (2017).

## Claims

**1.** An indium (III) phthalocyanine compound, which is 1,8(11),15(18),22(25)-tetra-(3,5-di-tert-butylphenoxy) indium (III) phthalocyanine, with the following formula for use in the treatment of cervical cancer;

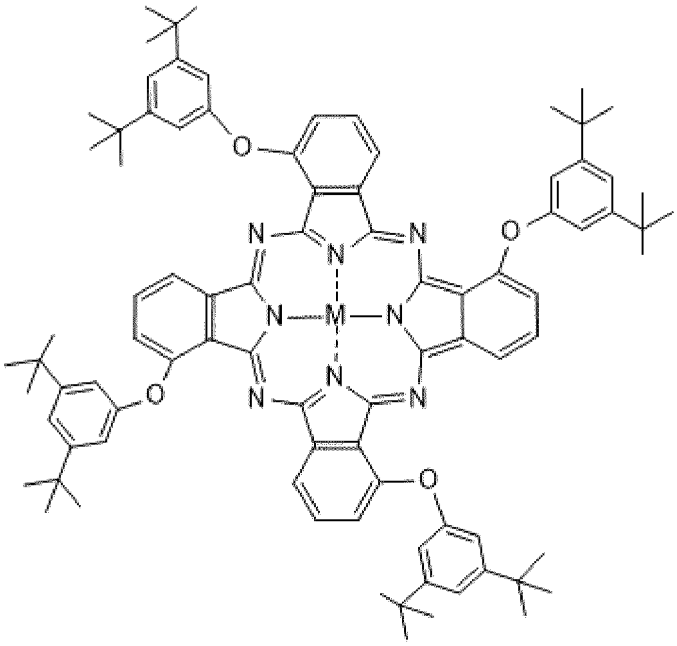

**Formula I**

2. A compound for use according to Claim 1, **characterized in that** the melting point thereof is higher than 300 °C.

3. A compound for use according to Claim 1, **characterized in that** said compound has a structure that is soluble in chloroform ($CHCl_3$), acetone ($C_3H_6O$), tetrahydrofuran (THF), ethanol ($C_2H_5OH$), methanol ($CH_3OH$), dimethylformamide (DMF) or dimethyl sulfoxide (DMSO).

4. A compound for use according to Claim 1, **characterized in that** after said compound is administered to the body, it causes the death of cancerous HeLa cells through apoptosis (programmed cell death) or necrosis when the cancerous region is exposed to red light.

5. An anticarcinogenic drug for use in the treatment of cervical cancer, comprising the compound according to any one of claims 1 to 4 as an active substance.

6. An indium (III) phthalocyanine compound, which is 1,8(11),15(18),22(25)-tetra-(3,5-di-tert-butylphenoxy) indium (III) phthalocyanine, with the following formula to be used in photodynamic therapy for the treatment of cervical cancer;

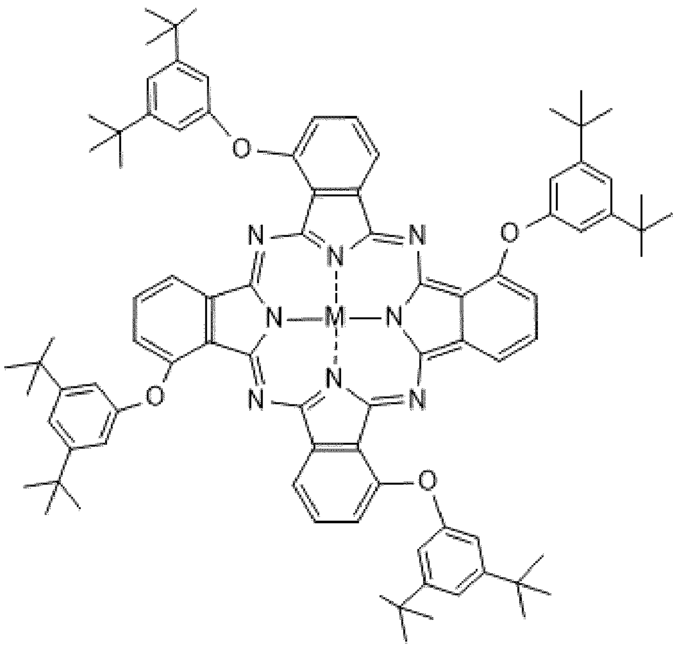

**Formula I**

7. A drug for use in the treatment of cervical cancer, comprising 1,8(11), 15(18),22(25)-tetra-(3,5-di-tert-butylphenoxy) indium (III) phthalocyanine compound.

8. 1,8(11),15(18),22(25)-tetra-(3,5-di-tert-butylphenoxy) indium (III) phthalocyanine compound for use in the preparation of a medicament with an anticarcinogenic effect for the treatment of cervical cancer.

9. A compound for use according to Claim 1 or Claim 6, **characterized in that** the photodegradation quantum yield thereof is $(\Phi_d) = 2{,}0017\times 10^{-05}$.

10. A compound for use according to Claim 1 or Claim 6, **characterized in that** 50 µM of compound concentration has a chemical structure that can reduce the cell viability to 78%, 100 µM of compound concentration has a chemical structure that can reduce the cell viability to 47,45% and 150 µM has a chemical structure that can reduce the cell viability to 28,81%.

11. A compound for use according to Claim 1 or Claim 6, having an antiproliferative effect on HeLa cells.

12. A compound for use according to Claim 1 or Claim 6, **characterized in that**; said compound has a chemical structure that reduces the mitosis index values from 6% to 3,7% at 24th hour; from 6,68% to 2,21% at 48th hour, and from 7,56% to 1,19% at 72nd hour with photodynamic therapy when applied to HeLa cells.

13. A compound for use in the treatment of cervical cancer according to Claim 1 or Claim 6, **characterized in that** said compound is administered through oral or intravenous route.

14. A compound for use in the treatment of cervical cancer according to Claim 1 or Claim 6, **characterized in that** said compound is in tablet form or in powder form to be rendered into a solution by means of a suitable solvent or in the form of a solution for injection.

**Patentansprüche**

1. Indium(III)-Phthalocyanin-Verbindung, die 1,8(11), 15(18),22(25)-Tetra-(3,5-di-tert-butylphenoxy)-Indium(III)-Phthalocyanin ist, mit der folgenden Formel zur Verwendung bei der Behandlung von Gebärmutterhalskrebs;

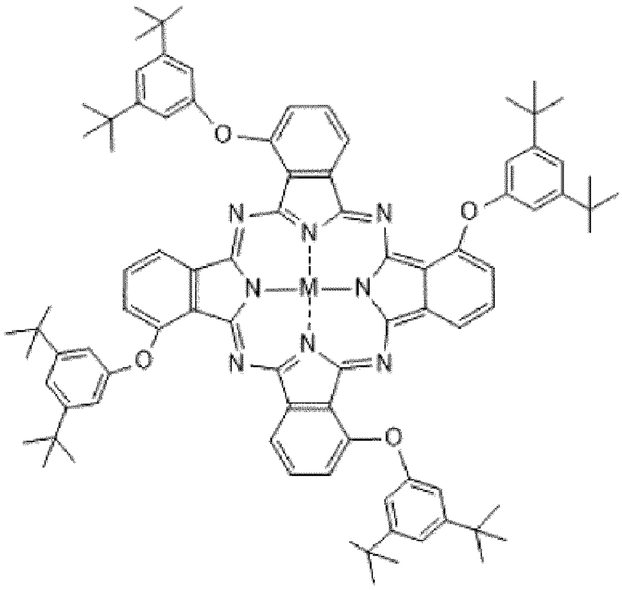

## Formel I

2. Verbindung zur Verwendung nach Anspruch 1, **dadurch gekennzeichnet, dass** ihr Schmelzpunkt höher als 300 °C ist.

3. Verbindung zur Verwendung nach Anspruch 1, **dadurch gekennzeichnet, dass** die Verbindung eine Struktur aufweist, die in Chloroform (CHCl3), Aceton (C3H6O), Tetrahydrofuran (THF), Ethanol (C2H5OH), Methanol (CH3OH), Dimethylformamid (DMF) oder Dimethylsulfoxid (DMSO) löslich ist.

4. Verbindung zur Verwendung nach Anspruch 1, **dadurch gekennzeichnet, dass** die Verbindung, nachdem sie dem Körper verabreicht wurde, den Tod von krebsartigen HeLa-Zellen durch Apoptose (programmierter Zelltod) oder Nekrose verursacht, wenn der krebsartige Bereich mit rotem Licht bestrahlt wird.

5. Anticarcinogenes Arzneimittel zur Verwendung bei der Behandlung von Gebärmutterhalskrebs, umfassend die Verbindung nach einem der Ansprüche 1 bis 4 als Wirkstoff.

6. Indium(III)-Phthalocyanin-Verbindung, die 1,8(11),15(18),22(25)-Tetra-(3,5-di-tert-butylphenoxy)-Indium(III)-Phthalocyanin ist, mit der folgenden Formel zur Verwendung in der photodynamischen Therapie zur Behandlung von Gebärmutterhalskrebs;

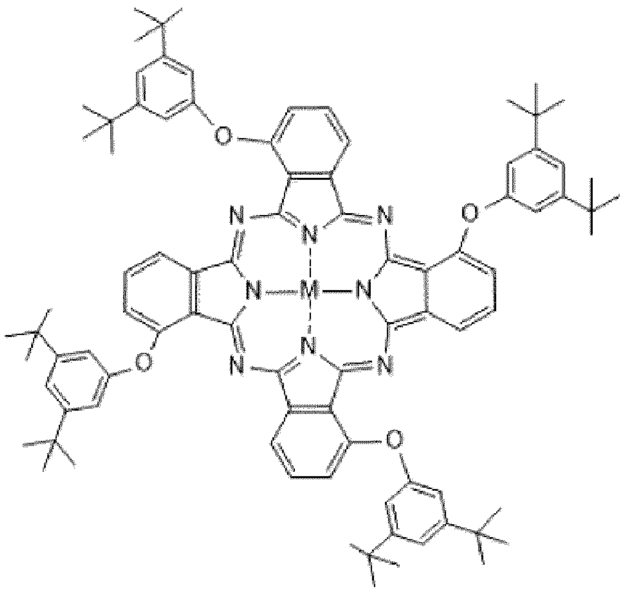

Formel I

7. Arzneimittel zur Verwendung bei der Behandlung von Gebärmutterhalskrebs, umfassend eine 1,8(11), 15(18),22(25)-Tetra-(3,5-di-tert-butylphenoxy)-Indium(III)-Phthalocyaninverbindung.

8. 1,8(11),15(18),22(25)-Tetra-(3,5-di-tert-butylphenoxy) Indium(III)-phthalocyanin-Verbindung zur Verwendung bei der Herstellung eines Arzneimittels mit antikarzinogener Wirkung zur Behandlung von Gebärmutterhalskrebs.

9. Verbindung zur Verwendung nach Anspruch 1 oder Anspruch 6, **dadurch gekennzeichnet, dass** ihre Photodegradationsquantenausbeute ($\Phi$d) = 2,0017$\times$10-$^{05}$ beträgt.

10. Verbindung zur Verwendung nach Anspruch 1 oder Anspruch 6, **dadurch gekennzeichnet, dass** 50 $\mu$M der Verbindungskonzentration eine chemische Struktur aufweist, die die Lebensfähigkeit der Zellen auf 78% reduzieren kann, 100 $\mu$M der Verbindungskonzentration eine chemische Struktur aufweist, die die Lebensfähigkeit der Zellen auf 47,45% reduzieren kann und 150 $\mu$M eine chemische Struktur aufweist, die die Lebensfähigkeit der Zellen auf 28,81% reduzieren kann.

11. Verbindung zur Verwendung nach Anspruch 1 oder 6, die eine antiproliferative Wirkung auf HeLa-Zellen hat.

12. Verbindung zur Verwendung nach Anspruch 1 oder Anspruch 6, **dadurch gekennzeichnet, dass** die Verbindung eine chemische Struktur aufweist, die die Mitoseindexwerte von 6 % auf 3,7 % in der 24. Stunde, von 6,68 % auf 2,21 % in der 48. Stunde und von 7,56 % auf 1,19 % in der 72.

13. Verbindung zur Verwendung bei der Behandlung von Gebärmutterhalskrebs nach Anspruch 1 oder Anspruch 6, **dadurch gekennzeichnet, dass** die Verbindung auf oralem oder intravenösem Wege verabreicht wird.

14. Verbindung zur Verwendung bei der Behandlung von Gebärmutterhalskrebs nach Anspruch 1 oder Anspruch 6, **dadurch gekennzeichnet, dass** die Verbindung in Tablettenform oder in Pulverform, die mittels eines geeigneten Lösungsmittels in eine Lösung zu überführen ist, oder in Form einer Lösung zur Injektion vorliegt.

**Revendications**

1. Composé de phtalocyanine d'indium (III), qui est la 1,8(11), 15(18), 22(25)-tétra-(3,5-di-tert-butylphénoxy) indium (III) phtalocyanine, avec la formule suivante pour l'utilisation dans le traitement du cancer du col de l'utérus ;

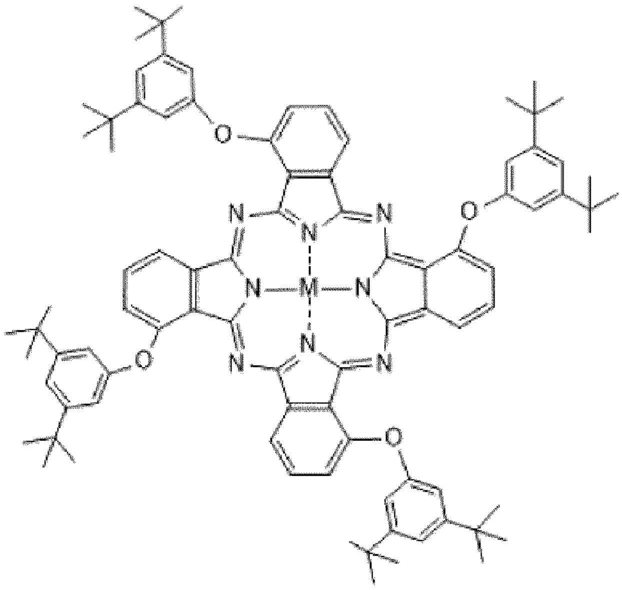

## Formule I

2. Composé pour être utilisé selon la revendication 1, **caractérisé en ce que** le point de fusion est supérieur à 300 °C.

3. Composé pour être utilisé selon la revendication 1, **caractérisé en ce que** ledit composé a une structure soluble dans le chloroforme ($CHCl_3$), l'acétone ($C_3H_6O$), le tétrahydrofurane (THF), l'éthanol ($C_2H_5OH$), le méthanol ($CH_3OH$), le diméthylformamide (DMF) ou le diméthylsulfoxyde (DMSO).

4. Composé pour être utilisé selon la revendication 1, **caractérisé en ce qu'**après administration dudit composé à l'organisme, il cause la mort des cellules cancéreuses HeLa par apoptose (mort cellulaire programmée) ou nécrose lorsque la région cancéreuse est exposée à la lumière rouge.

5. Médicament anticancéreux pour être utilisé dans le traitement du cancer du col de l'utérus, comprenant le composé selon l'une quelconque des revendications 1 à 4 comme substance active.

6. Composé de phtalocyanine d'indium (III), qui est la 1,8(11),15(18),22(25)-tétra-(3,5-di-tert-butylphénoxy) indium (III) phtalocyanine, avec la formule suivante à utiliser dans la thérapie photodynamique pour le traitement du cancer du col de l'utérus ;

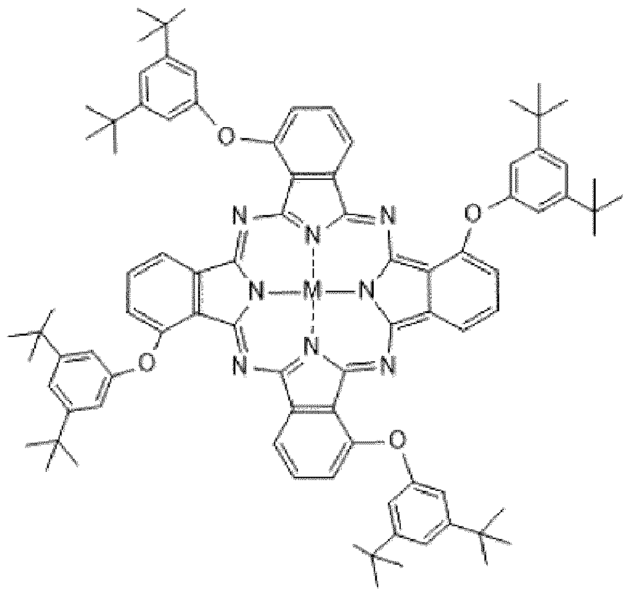

**Formule I**

7. Médicament pour être utilisé dans le traitement du cancer du col de l'utérus, comprenant le composé 1,8(11), 15(18), 22(25)-tétra-(3,5-di-tert-butylphénoxy) indium (III) phtalocyanine.

8. Composé de 1,8(11),15(18),22(25)-tétra-(3,5-di-tert-butylphénoxy) indium (III) phtalocyanine pour être utilisé dans la préparation d'un médicament à effet anticancéreux pour le traitement du cancer du col de l'utérus.

9. Composé pour être utilisé selon la revendication 1 ou la revendication 6, **caractérisé en ce que** le rendement quantique de photodégradation est (Φd) = 2,0017x10-05.

10. Composé pour être utilisé selon la revendication 1 ou la revendication 6, **caractérisé en ce que** 50 μM de concentration de composé a une structure chimique pouvant réduire la viabilité cellulaire à 78%, 100 μM de concentration de composé a une structure chimique pouvant réduire la viabilité cellulaire à 47,45% et 150 μM a une structure chimique pouvant réduire la viabilité cellulaire à 28,81%.

11. Composé pour être utilisé selon la revendication 1 ou la revendication 6, ayant un effet antiprolifératif sur les cellules HeLa.

12. Composé pour être utilisé selon la revendication 1 ou la revendication 6, **caractérisé en ce que** ledit composé a une structure chimique réduisant les valeurs de l'indice de mitose de 6% à 3,7% à la $24^{e}$ heure ; de 6,68% à 2,21% à la $48^{e}$ heure et de 7,56% à 1,19% à la $72^{e}$ heure avec une thérapie photodynamique lorsqu'il est appliqué aux cellules HeLa.

13. Composé pour être utilisé dans le traitement du cancer du col de l'utérus selon la revendication 1 ou la revendication 6, **caractérisé en ce que** ledit composé est administré par voie orale ou intraveineuse.

14. Composé pour être utilisé dans le traitement du cancer du col de l'utérus selon la revendication 1 ou la revendication 6, **caractérisé en ce que** ledit composé est sous forme de comprimé ou sous forme de poudre à mettre en solution au moyen d'un solvant approprié ou sous forme de solution injectable.

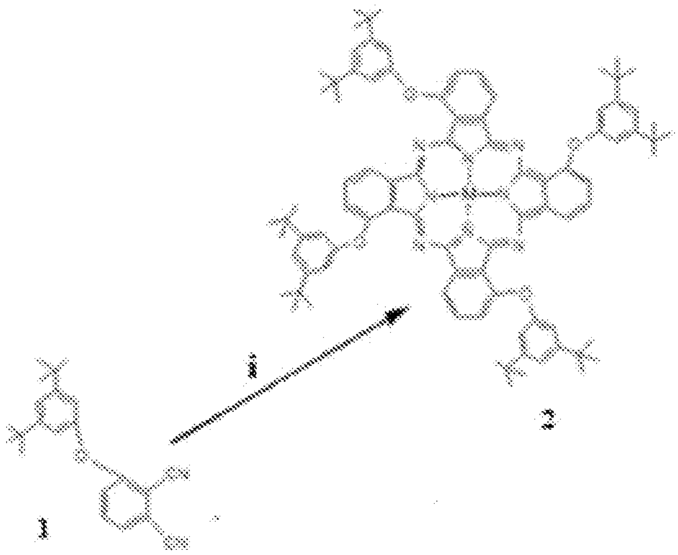


1

2

M: In

FIGURE 1

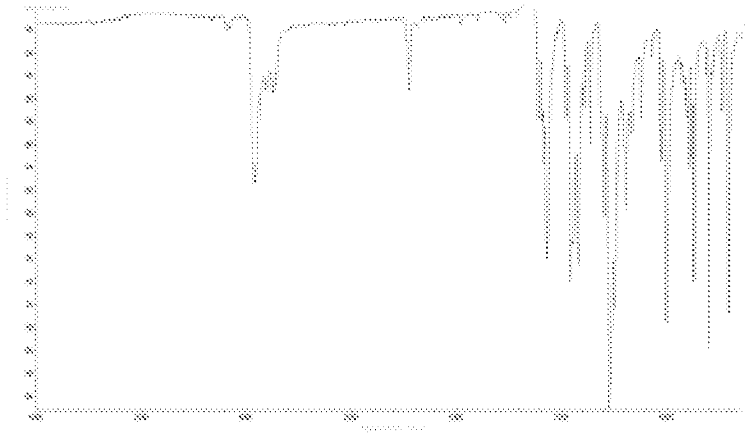

FIGURE 2

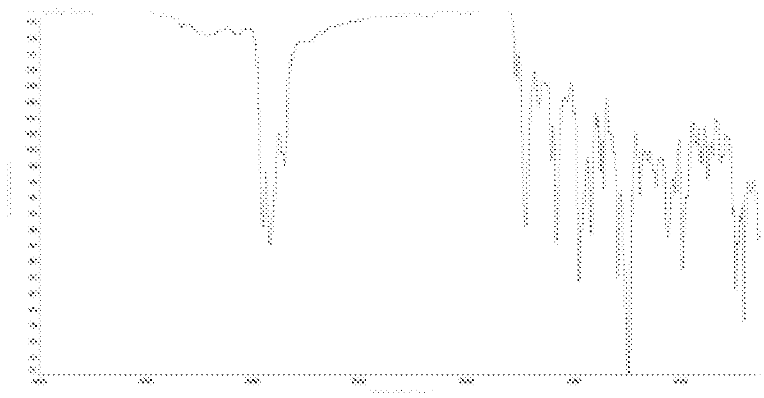

FIGURE 3

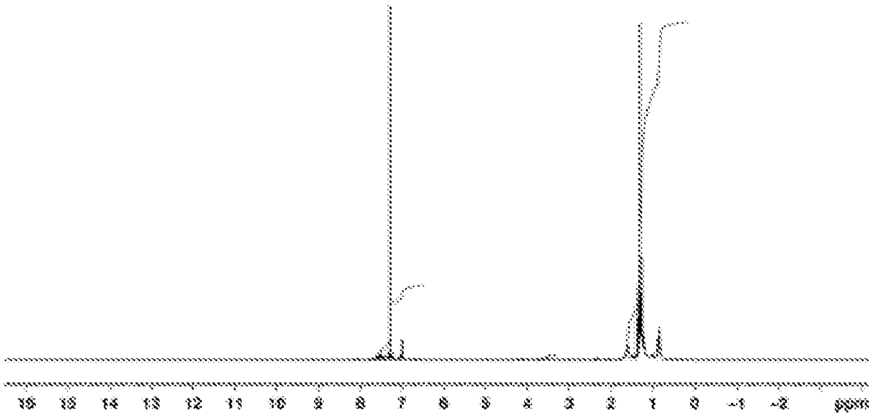


FIGURE 4

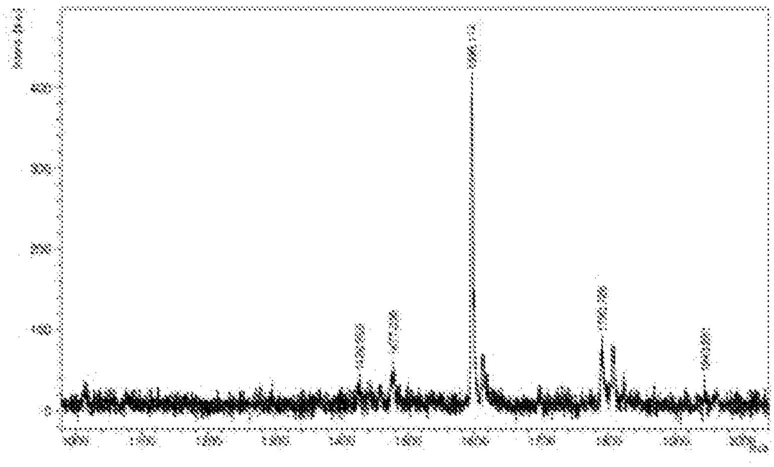

FIGURE 5

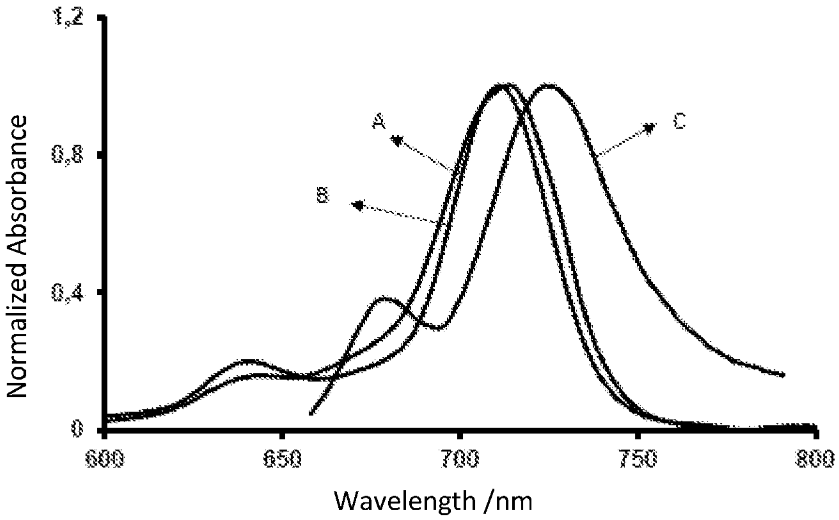


**FIGURE 6**

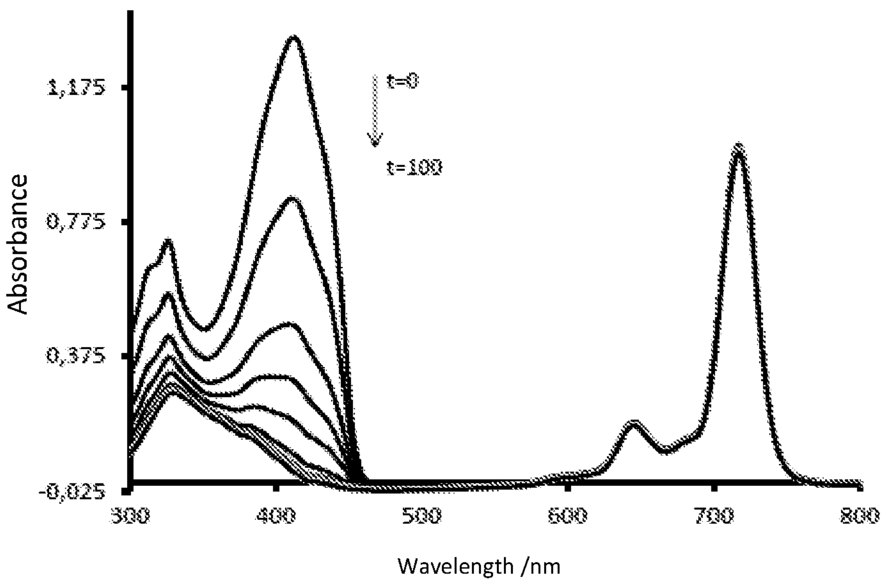


**FIGURE 7**

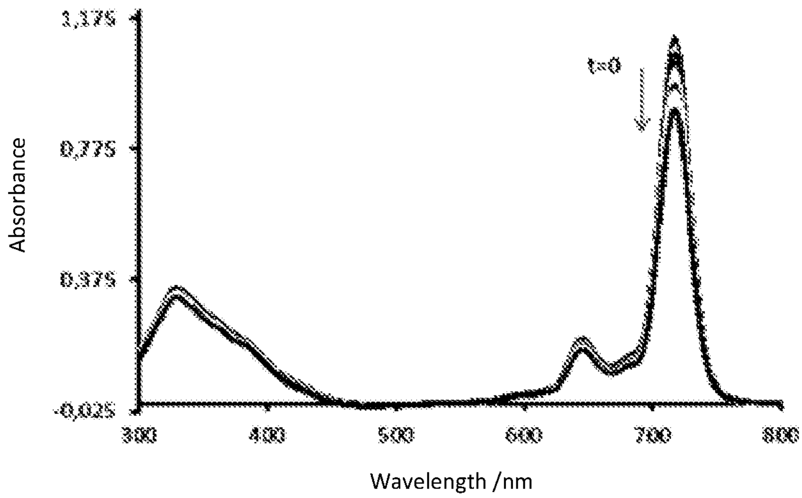
1,175
0,775
0,375
-0,025
Absorbance
t=0
300
400
500
600
700
800
Wavelength /nm

FIGURE 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **KLUG, W.S** ; **CUMMINGS, M.R** ; **SPENCER, C.A.** Concepts of Genetics. Pearson, 2006, 13191883-8
- Some basic and applied principles of cancer chemotherapy. **PITOT HC** ; **LOEB DD**. Fundamentals of Oncology. Marcel Dekker Inc, 2002, 901-945
- **WU HC** ; **HUANG CT** ; **CHANG DK**. Anti-angiogenic therapeutic drugs for treatment of human cancer. *J Cancer Molecules*, 2008, vol. 4, 37-45
- Environmental, genetic, and viral causes of cancer. **AQEILAN RI** ; **ZANESI N** ; **CROCE CM**. The biology and treatment of cancer. John Wiley & Sons, Inc, 2009, 35-56
- **M. BREWIS** ; **G.J. CLARKSON** ; **P. HUMBERSTONE** ; **S. MAKHSEED** ; **N.B. MCKEOWN**. The synthesis of some phthalocyanines and napthalocyanines derived from sterically hindered phenols. *Chem. Eur. J.*, 1998, vol. 4, 1633-1640
- **S. FERY-FORGUES** ; **D. J. LAVABRE**. Are fluorescence quantum yields so tricky to measure? A demonstration using familiar stationery products. 1999, vol. 76, 1260-1264
- Encapsulation of phthalocyanines in biodegradable poly (sebacic anhydride) nanoparticles. **J. FU** ; **X.Y. LI** ; **D.K.P. NG** ; **C. WU**. Langmuir. Ed. 2002, vol. 18, 3843-3847
- **A. OGUNSIPE** ; **A.D. MAREE** ; **T.J. NYOKONG**. Solvent effects on the photochemical and fluorescence properties of zinc phthalocyanine derivatives. *Mol Struct*, 2003, vol. 650, 131-140
- **A. OGUNSIPE** ; **J.Y. CHEN** ; **T. NYOKONG**. Photophysical and photochemical studies of zinc (II) phthalocyanine derivatives-effects of substituents and solvents. *New J. Chem*, 2004, vol. 28, 822-827
- **D. WOHRLE** ; **G. SCHNURPFEIL** ; **S.G. MAKAROV** ; **A. KAZARIN** ; **O.N. SUVOROVAB**. Practical applications of phthalocyanines-from dyes and pigments to materials for optical, electronic and photo-electronic devices. *Macroheterocycles*, 2012, vol. 5, 191-202
- **M.O. LIUA** ; **C. TAIB** ; **M. SAINA** ; **A. TEH HUA** ; **F. CHOUC**. Photodynamic applications of phthalocyanines. *J. Photochem. Photobiol. A Chem*, 2004, vol. 165, 131-136
- **I. J. MACDONALD** ; **T.J. DOUGHERTY**. Basic principles of photodynamic therapy. *J. Porphyrins Phthalocyan*, 2001, vol. 5, 105-129
- **J. E. VAN LIER** ; **N. BRASSEUR** ; **B. PAQUETTE** ; **J. R. WAGNER** ; **H. ALI** ; **R. LANGLOIS** ; **J. ROUSSEAU**. Phthalocyanines as Sensitizers for Photodynamic Therapy of Cancer. *NATO ASI Series*, vol. H15
- **SHAWN SWAVEY** ; **MATTHEW TRAN**. Porphyrin and Phthalocyanine Photosensitizers as PDT Agents: A New Modality for the Treatment of Melanoma, Recent Advances in the Biology. *Therapy and Management of Melanoma*, 2013
- **VROUENRAETS MB** ; **VISSER GW** ; **SNOW GB** ; **VAN DONGEN GA**. Basic principles, applications in oncology and improved selectivity of photodynamic therapy. *Anticancer Research*, 2003, vol. 23 (1), 505-522
- **DOLMANS DE** ; **FUKUMURA D** ; **JAIN RK**. Photodynamic therapy for cancer. *Nature Reviews Cancer*, 2003, vol. 3 (5), 380-387
- **HODGKINSON N** ; **KRUGER CA** ; **MOKWENA M** ; **ABRAHAMSE H.** Cervical cancer cells (HeLa) response to photodynamic therapy using a zinc phthalocyanine photosensitizer.. *Journal of Photochemistry & Photobiology*, 2017, vol. 177, 32-38
- **GUAN D** ; **GUO Q** ; **CHEN L** ; **WU S** ; **NAN F** ; **ZHANG Y**. Study on application of photodynamic therapy for the treatment of cervical cancer.. *Int J Clin Exp Med*, 2016, vol. 9 (12), 23337-23344
- **LI W** ; **TAN G** ; **CHENG J** ; **ZHAO L** ; **WANG Z** ; **JIN Y.** A Novel Photosensitizer 31,131-phenylhydrazine -Mppa (BPHM) and Its in Vitro Photodynamic Therapy against HeLa Cells. *Molecules*, 2016, vol. 21 (5), 558
- **MASCARAQUE M** ; **DELGADO-WICKE P** ; **DAMIAN A** ; **LUCENA SR** ; **CARRASCO E** ; **JUARRANZ A.** Mitotic Catastrophe Induced in HeLa Tumor Cells by Photodynamic Therapy with Methyl-aminolevulinate. *Int. J. Mol. Sci.*, 2019, vol. 20, 1229
- **KOCAN, H.** ; **KAYA, K.** ; **OZCESMECI, I. et al.** Photophysicochemical, calf thymus DNA binding and in vitro photocytotoxicity properties of tetra-morpholinoethoxy-substituted phthalocyanines and their water-soluble quaternized derivatives.. *J Biol Inorg Chem*, 2017, vol. 22, 1251-1266